# EUROPEAN PATENT SPECIFICATION

(11) **EP 4 243 699 B1**
(45) Date of publication and mention of the grant of the patent: **22.04.2026**
(21) Application number: 21819685.5
(22) Date of filing: 11.11.2021
(51) Int. Cl.: A61B 17/00, A61B 17/12, A61B 17/04

(54) **WIRE ASSEMBLIES FOR BLOOD VESSEL OCCLUSION IN VASCULAR PROCEDURES**
DRAHTANORDNUNGEN FÜR BLUTGEFÄSSOKKLUSION BEI GEFÄSSEINGRIFFEN
ENSEMBLES DE FILS D'OCCLUSION DE VAISSEAU SANGUIN DANS DES INTERVENTIONS VASCULAIRES

(30) Priority: 13.11.2020 US 202063113427 P
(43) Date of publication of application: 20.09.2023
(73) Proprietor: Teleflex Life Sciences LLC, Wilmington, DE 19808 (US)
(72) Inventor: BULLER, Christopher, E., Minneapolis, Minnesota 55369 (US); BRENIZER, Joshua, Minneapolis, Minnesota 55369 (US); GRINTZ, Joseph, Todd, Wayne, Pennsylvania 19087 (US)
(74) Representative: Haseltine Lake Kempner LLP
(86) International application number: PCT/US2021/058905
(87) International publication number: WO 2022/103921

(56) References cited:
- WO-A1-2009/126747
- WO-A2-2006/115904
- US-A1- 2005 245 894
- US-A1- 2014 194 918
- US-B2- 7 331 979

## Description

### TECHNICAL FIELD

The present disclosure relates to medical devices.

### BACKGROUND

During vascular procedures, sheath exchange or removal can lead to a significant loss of blood. One way this may be mitigated is to utilize temporary occlusion of the vessel proximal to the sheath site. Occlusion creates flow stasis, allowing for bloodless sheath removal or exchange. For example, U.S. Patent Publication No. 20140194918A1 is directed to a blood flow cessation device that includes a catheter, an expandable member, and a rapid exchange port. The catheter includes a distal end portion and a proximal end portion. The expandable member is positioned at the distal end portion and is selectively expandable to stop blood flow through a vessel.

However, current occlusion assemblies make it difficult to manage flow while allowing additional devices into the vessel for various procedures.

### SUMMARY

The wire assembly of the present invention is defined in claim 1. Further embodiments of the invention are recited in the dependent claims. An embodiment of the present disclosure is a method that includes inserting a wire assembly through an opening in a vessel so that a distal end portion of the wire assembly is positioned inside the vessel. The wire assembly has a shaft, an occlusion member disposed along a distal end portion of the shaft, and a proximal end portion spaced from the distal end portion along a longitudinal axis. The wire assembly further having cross-sectional dimensions along its length that are perpendicular to the longitudinal axis. The method includes expanding the occlusion member from a collapsed configuration into an expanded configuration such that the occlusion member occludes blood flow in the vessel while the distal end portion of the wire assembly is positioned inside the vessel. The method includes causing the occlusion member to transition from the expanded configuration into the collapsed configuration so that in the collapsed configuration, the occlusion member may have a cross-sectional dimension relative to a cross-sectional dimension of the shaft that does not substantially inhibit movement of a device along the occlusion member or the shaft. Alternatively, the method may include movement and removal of the wire assembly through a fixed device containing a wire lumen or orifice of a diameter compatible with the wire component of the wire assembly.

### BRIEF DESCRIPTION OF THE DRAWINGS

The foregoing summary, as well as the following detailed description, will be better understood when read in conjunction with the appended drawings. The drawings show illustrative embodiments of the disclosure. It should be understood, however, that the application is not limited to the precise arrangements and instrumentalities shown.
Figure 1A is a plan view of a wire assembly in accordance with an embodiment of the present disclosure;
Figure 1B is a plan view of the wire assembly shown in Figure 1A;
Figure 2A is a perspective view of an occlusion member according to an embodiment of the present disclosure;
Figure 2B is a perspective view of the occlusion member shown in Figure 2A;
Figure 3A is a side view of an occlusion member according to an embodiment of the present disclosure; and
Figure 3B is an end view of the occlusion member shown in Figure 3A.
Figure 4 is a perspective view of the vascular closure device according to an embodiment of the present disclosure;
Figure 5 is a perspective view of a sealing device associated with the vascular closure device shown in FIG. 4; and
Figure 6 is a side sectional view of a distal portion of the vascular closure device.

### DETAILED DESCRIPTION OF ILLUSTRATIVE EMBODIMENTS

Certain terminology is used in the following description for convenience only and is not limiting. The words "right", "left", "lower" and "upper" designate directions in the drawings to which reference is made. The words "proximally" and "distally" refer to directions toward and away from, respectively, the individual operating the system. The terminology includes the above-listed words, derivatives thereof and words of similar import.

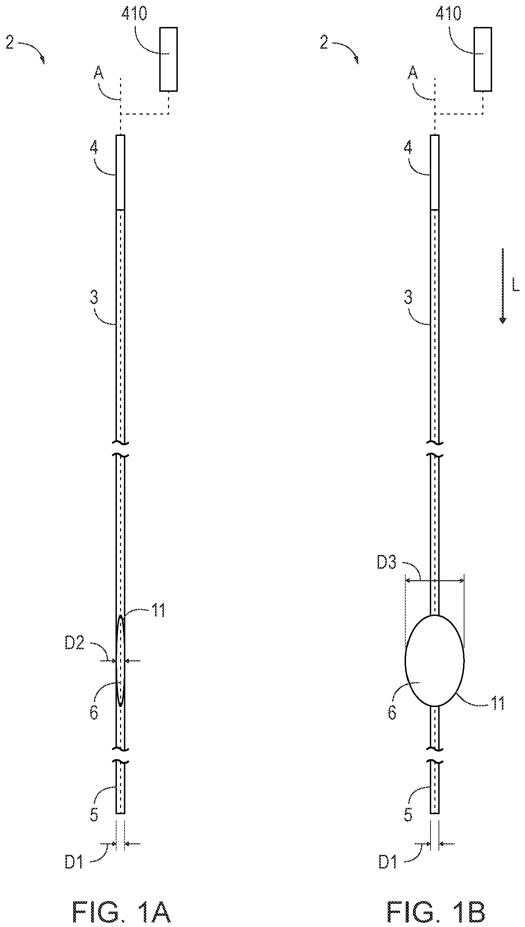

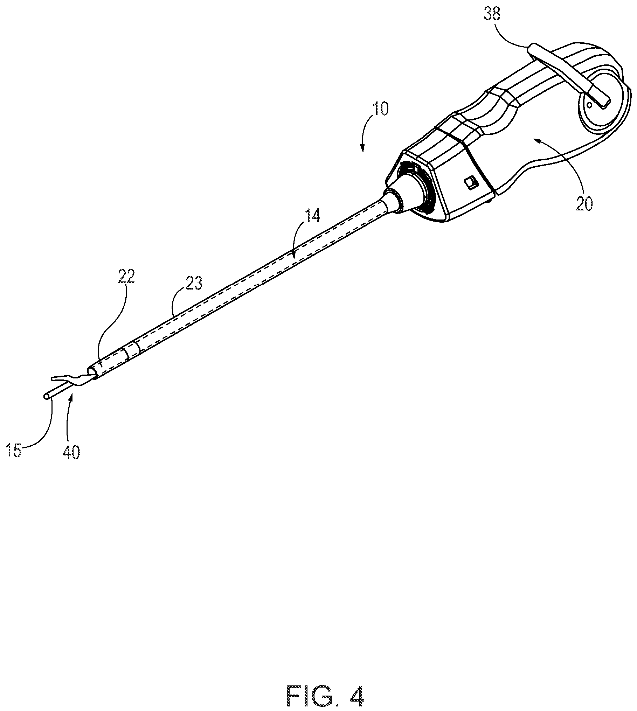

Referring to FIGS. 1A-3B, the present disclosure include embodiments of a wire assembly 2, 102, 202 that may be used to manage blood flow in a vessel, e.g. an artery or vein, during vascular procedures. More specifically, the wire assembly 2, 102, 202 is configured to selectively occlude blood flow, as will be described further below. The wire assembly 2, 102, 202 is further configured to minimize dilation of the vessel or the opening through which the wire assembly 2, 102, 202 was inserted into the vessel during use. In addition, the wire assembly 2, 102, 202 permit deployment of other devices over and along the wire assembly and to also allow the wire assembly to be withdrawn from the deployed devices without significantly altering or impeding use of such other devices. For instance, the wire assembly 2, 102, 202 is configured such that other surgical devices may slide along the wire assembly. Likewise, the wire assembly 2, 102, 202 is configured so that it may be inserted or guided through surgical devices, such as, for example, inserted through a vascular closure device 12 (Figure 4).

As shown in FIGS. 1A-1B, an embodiment wire assembly 2 is shown that includes a shaft 4 and an occlusion member 6. In one example, the shaft 4 of the wire assembly 2 may be a guide wire. As shown, the shaft 4 is elongated along a longitudinal axis A that in turn extends along a longitudinal direction L. The shaft 4 has a proximal end portion 3, a distal end portion 5 spaced from the proximal end portion 3 along the longitudinal axis A, and a lumen that extends from the proximal end portion 3 toward the distal end portion 5. The distal end portion 5 of the shaft 4 may include a step-down region (not shown or numbered) and may also have, as needed, a preformed J-shape or pigtail shape.

As shown, the shaft 4 also has a cross-sectional dimension D1 that is perpendicular to the longitudinal axis A. In the illustrated embodiment, the cross-sectional dimension D1 is no greater than about 0.040 in (0.1016 cm). For example, the cross-sectional dimension D1 may be 0.014 in (0.0356 cm). In another example, the cross-sectional dimension D1 may be 0.018 in (0.0457 cm). In another example, the cross-sectional dimension D1 may be 0.035 in (0.0889 cm). In another embodiment, the cross-sectional dimension D1 may vary along the longitudinal axis A.

Continuing with Figure 1A and 1B, the occlusion member 6 is positioned along the shaft 4. The occlusion member 6 is configured to transition between a collapsed configuration, where the occlusion member 6 is contracted for insertion into the vessel, and an expanded configuration, where occlusion member occludes blood flow when in the vessel. The occlusion member 6 in the collapsed configuration has a cross-sectional dimension D2, which is perpendicular to the axis A as shown. When in the expanded configuration, the occlusion member has a cross-sectional dimension D3 that is greater than the cross-sectional dimension D2. In the collapsed configuration, the occlusion member cross-sectional dimension D2, relative to a cross-sectional dimension D1 of the shaft 4, does not substantially inhibit movement of a device to move along the occlusion member 6 or the shaft 4. The relative similarity in cross-sectional dimension D1 of the shaft 4 and the cross-sectional dimension D2 of the occlusion member when collapsed facilitates movement of devices along the wire assembly without catching such devices or otherwise causing damage to such devices, or alternatively, the movement and removal of the wire assembly through a fixed device with a wire lumen or orifice compatible with the wire component of the wire assembly. In this configuration, such a wire assembly can be used to occlude blood flow while permitting device use flexibility during a procedure. In one example, the cross-sectional dimension D2 of the occlusion member when collapsed may be substantially no greater than the cross-sectional dimension D1 of the shaft 4. In the illustrated embodiment, the cross-sectional dimension D2 of the occlusion member 6 is no greater than about 0.035in (0.0889 cm)., such as up to about 0.040 in (0.1016 cm). In another embodiment, the cross-sectional dimension D2 of the occlusion member 6 may vary. For example, the cross-sectional dimension D2 may be no more than any one of about 0.014in (0.0356 cm), 0.018in (0.0457 cm), or 0.035in (0.0889 cm). It should be appreciated that the cross-sectional dimension D2 can vary beyond those values cited in this disclosure. Furthermore, the occlusion member 6 is sized and configured such that when it is expanded, it does not dilate a vessel into which it is inserted. In addition, when the occlusion member 6 is collapsed it does not dilate the puncture hole of the vessel that provides access into the vessel.

As illustrated, the occlusion member 6 is positioned at a distal tip of the shaft 4. Where a step-down region is present in the shaft 4, the occlusion member 6 may be disposed along the step-down region 9 of the shaft 4 that is proximal to the distal tip. The occlusion member 6 can be positioned, however, along any portion of the shaft configured to be inserted into the vessel.

The wire assembly 2 may include a removable hub 410. Specifically, the removeable hub 410 may be configured to be connected to the proximal end portion 3 of the shaft 4. The hub 410 may be used to couple to an external device, such as fluid control device or actuator, as will be explained below.

The removable hub 410 and/or the shaft 4 may include a connection member (not shown or numbered) used to couple the removable hub 410 to the proximal end portion 3 of the shaft 4. The connection member may have a cross-sectional diameter that is substantially the same or less than the cross-section diameter D1 of the shaft 4, so that other devices may be delivered over the connection. Thus, in one embodiment, the connection member has a cross-sectional dimension that is not substantially greater than the cross-sectional dimension D1 of the shaft 4.

In one example, the wire assembly may include a connection member (not numbered) that is configured to couple the shaft 4 to the hub 410. In one example, the connection member is threaded helically such that the hub 410 can be threadably coupled to the shaft 4. The proximal end portion 3 of the shaft 4 can be externally threaded to mate with internal threads in the removable hub 410, and the removable hub 410 can be removed from the shaft 4 by rotating the removable hub 410 relative to the shaft 4. In another example, the connection member is a press-fit or fluid tight connection. In such an example, the press fit connection may include a soft rubber seal or mechanical features configured to engage with each other via slight pressure by the user. In yet another example, the connection between the removable hub 410 and the proximal end portion 3 of the shaft 4 may include a barbed fitting that is configured to be engaged and disengaged.

In one embodiment, the wire assembly 2 further includes a valve (not shown) disposed in the lumen of the shaft 4. The valve is configured to permit the occlusion member 6 to maintain the transition from the collapsed configuration to the expanded configuration. The valve may be similar to a hemostasis valve or a touhey borst adaptor or valve. In some cases, the valve may include the removable hub 410.

In the embodiment shown in Figures 1A and 1B, the occlusion member 6 is an expandable balloon comprised of a compliant membrane 11 that is configured to be inflated and deflated. The balloon may be manufactured from any suitable elastomeric rubber-like compound such as natural rubber latex, isoprene, neoprene, butyl rubber, or silicone. In other embodiments, various occlusion members may be utilized as described below. The occlusion member 6 may be attached to the shaft 4 via any number of mechanisms. For example, the compliant membrane 11 is wrapped around and fused into the shaft 4. While a compliant membrane 11 is disclosed, the occlusion member 6 may be formed from a non-compliant membrane.

In the illustrated embodiment, a user activates the occlusion member 6 by fully or partially inflating or deflating the occlusion member 6 via a fluid control device (not shown). The fluid control device as used herein may be configured to both inflate and deflate the occlusion member as needed. The removable hub 410 can be further coupled to a fluid control device to inflate the occlusion member 6.

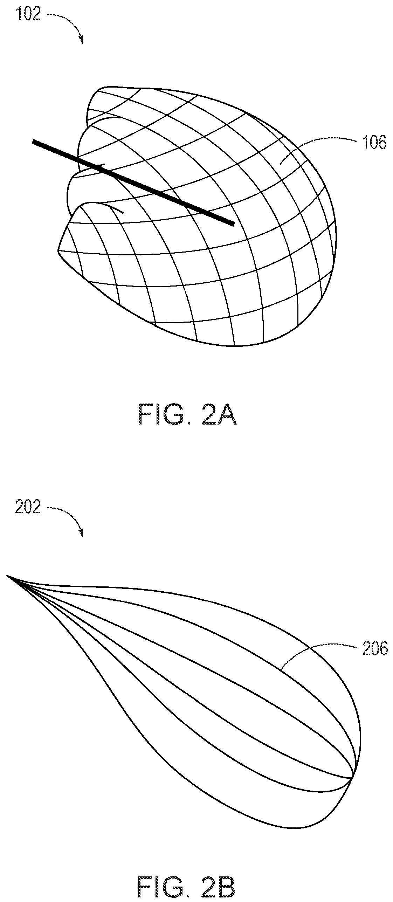

Turning to Figures 2A and 2B, alternative configurations of an occlusion member are shown. In Figure 2A, the occlusion member 106 includes another version of a balloon structure that may be used to occlude blood flow. The occlusion member 106 shown in Figure 2A may include an expandable membrane configured to expand when a fluid is inserted therein. Thus, the occlusion member 106 can transition between a collapsed configuration and an expanded configuration (as shown). The occlusion member 106 as shown in Figure 2A may include any or all of the features of the wire assembly 2 described herein. In Figure 2B, the occlusion member 206 is shown as another structure that includes a basket coupled to the shaft, and an optional membrane disposed around the basket to facilitate occlusion when inserted in the vessel. The occlusion member 206 shown in Figure 2B may also transition between a collapsed configuration and an expanded configuration (as shown). The occlusion member 206 as shown in Figure 2B may include any or all of the features of the wire assembly 2 described herein.

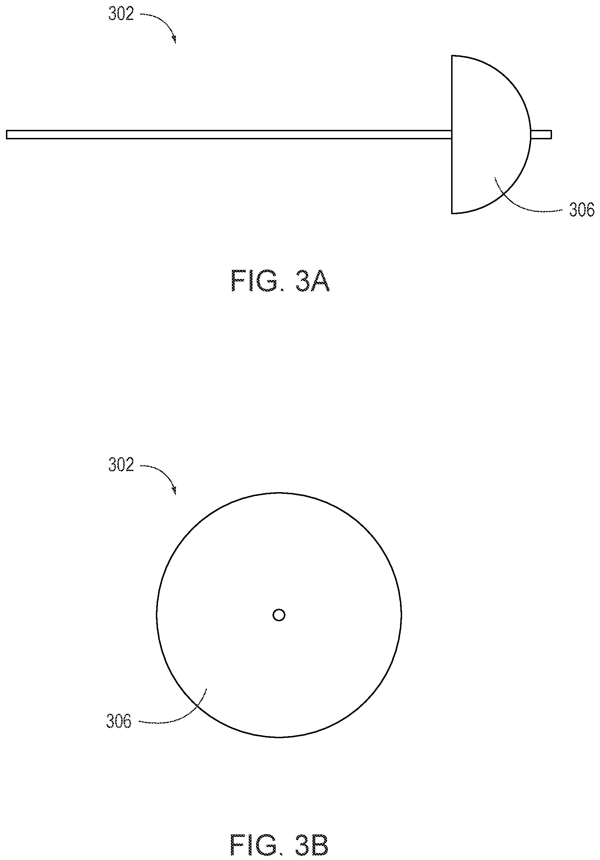

Referring to Figures 3A and 3B, a wire assembly 302 according to another embodiment of the present disclosure is illustrated. As shown, the occlusion member 306 is positioned on the wire assembly 2 and may be an expandable dam 306 configured to occlude blood flow. The expandable dam 306 may be a dome, umbrella or similar shape that is configured to transition between a collapsed configuration and an expanded configuration. In another example, the expandable dam 306 may be a nitinol structure that is expanded.

In one embodiment, the wire assembly 302 may include an actuator (not shown) configured to transition the occlusion member 306 between the collapsed configuration and the expanded configuration. The actuator may be any device configured to cause the expandable dam 306 to expand and collapse. In one example, the actuator includes a tube (not shown) disposed around and moveable relative to the shaft. In such an example, in the collapsed configuration, the tube contains the expandable dam 306, and in the expanded configuration the expandable dam 306 is released from the tube such that expandable dam 306 expands. In another example, the expandable dam 306 may be formed in part of a nitinol basket. In such an example, the actuator includes a wire coupled to the nitinol basket. Actuation of the actuator pulls the wire in proximal direction causing the nitinol basket to 1) compress along the longitudinal axis and 2) expand along a radial direction that is angularly offset with respect to the longitudinal axis. The wire or other elongated element may be configured to push or pull the tube as needed. In embodiments where an actuator is used, the occlusion member 306 may still be partially expanded and partially collapsed as needed.

The wire assembly as described herein may be used with a variety of surgical or vascular devices. For purposes of illustrating the embodiments of the disclosure, the wire assembly is described in connection with a vascular closure device. It is clear, however, that devices other than the vascular closure device specifically described herein can be used with the wire assembly. Accordingly, referring to Figures 4-6, in the illustrated embodiment, the wire assembly described herein is used in connection with a vascular closure device 12 for occlusion during vascular procedures. In alternative embodiments, the wire assembly 2 described herein may be used in combination with various devices for any vascular procedure. Turning to Figures 4-7, a vascular closure device 12 is shown that is configured to seal a puncture in a vessel wall. The closure device 12 includes a deployment assembly 14 and an access sheath 23. The access sheath 23 includes a hub and shaft (not numbered). The access sheath 23 can be inserted into the vessel and the deployment assembly 14 can be inserted into the access sheath 23 to position a sealing unit 18 into the vessel. The closure device 12 may include a guide lumen 15 (Figure 4) that extends through an entirety of the closure device 12. The closure device 12 can be inserted along the wire assembly and will be described further below.

Referring to Figures 4-6, the vascular closure device 12 includes a sealing unit 18 at least partially disposed within a deployment assembly 14. The vascular closure device 12 can be configured such that after a distal portion of deployment assembly 14 is inserted through a puncture site of the vessel, the sealing unit 18 is deployed to thereby seal or otherwise close the puncture site of the vessel. The deployment assembly 14 is configured to control orientation of a toggle or anchor 40 of the sealing unit 18 in an easier and more efficient manner during deployment of the sealing unit 18. Furthermore, the deployment assembly 14 is configured to reduce forces required to deploy the sealing unit 18 and seal the puncture.

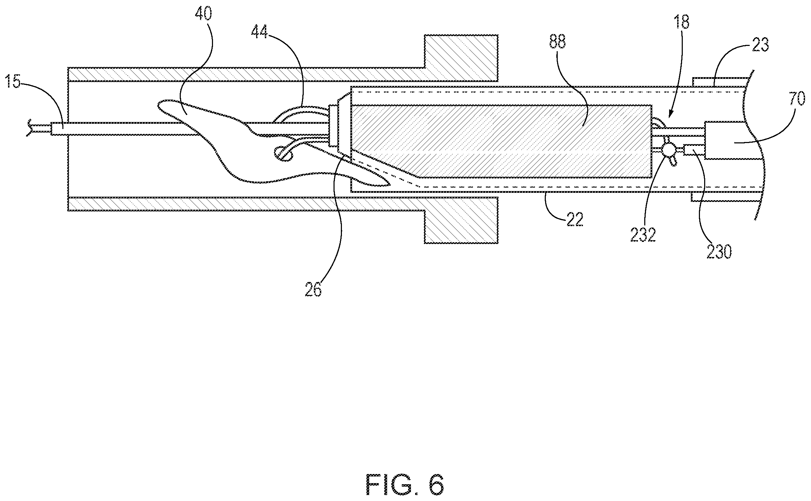

In accordance with the illustrated embodiment, the deployment assembly 14 includes a release component 22 that restrains the toggle 40, a delivery component 26 (see Figure 4B) that contains at least a portion of the toggle 40 and a suture 43 of the sealing unit 18, a guide member 35, and one or more actuators or release knobs 38 coupled to the release component 22. The deployment assembly 14 may also include a tamper 70, in the form a tube, that extends along the suture 43 and is located in a proximal direction relative to the locking member 230 (see Figure 6). The guide member 35 extends through the sealing unit 18 and is configured to receive a wire as will be discussed below. In another example, the deployment assembly 14 can be configured so that the wire extends along the side of the toggle 40. The release component 22 is operatively associated with the suture 43 such that actuation of the release knob 38 causes the release component 22 to 1) release the toggle 40, and 2) apply tension to the suture 43, which urges the toggle 40 against the delivery component 26 and orients the toggle 40 in the sealing position. The guide member 35 is configured to be removed from at least the sealing unit 18 prior the sealing unit 18 sealing the puncture.

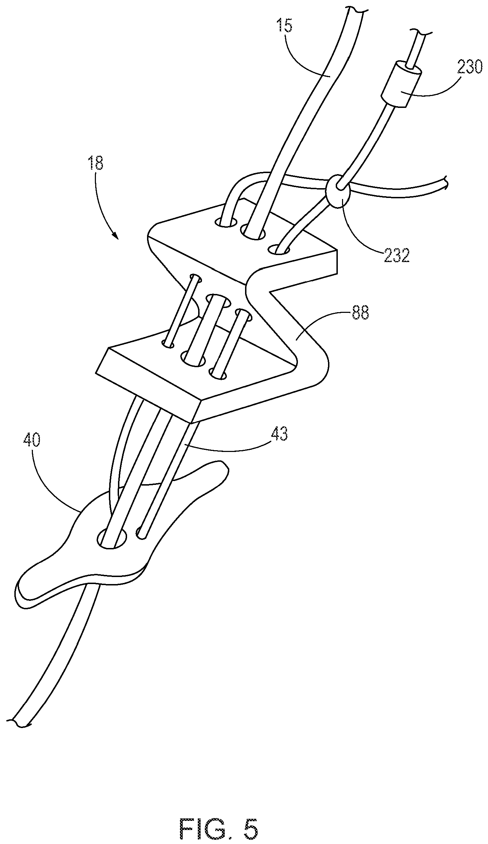

Continuing with to Figures 5-6, the sealing unit 18 includes the toggle 40 connected to the suture 43, a plug 88 coupled to the suture 43 and spaced from the toggle 40 in a proximal direction, and a locking member 230 proximal to the plug 88. The toggle 40 includes a distal end and a proximal end opposite to the proximal end, and a plurality of apertures extending therethrough. The suture 43 extends through the apertures as illustrated such that an end of the suture 43 is formed into a slidable knot 232. The knot 232 is slidable along the suture 43 between the plug 88 and the locking member 230. In an implanted state, the toggle 40 is adjacent to an inner surface of the vessel and the locking member 230 squeezes the toggle 40 and the plug 88 against the vessel to seal the puncture.

The sealing unit 18 is formed with materials suitable for surgical procedures such as any biocompatible material. It should be appreciated, however, that the toggle 40 can be made of other materials and can have other configurations so long as it can be seated inside the vessel against the vessel wall. The plug 88 may comprise a strip of compressible, resorbable, collagen foam and may be made of a fibrous collagen mix of insoluble and soluble collagen that is cross linked for strength. It should be appreciated, however, that the plug member 88 may have any configuration as desired and can be made from any material as desired. The suture 43 may be any elongate member, such as, for example a filament, thread, or braid.

Embodiments of the present technology will now be described with respect to exemplary methods, such as vascular procedures, that utilize the wire assemblies described herein. In order to perform any of the related procedures, the user gains percutaneous access to a vessel, which may be an artery or a vein. Reference to an artery below does not exclude use of the wire assembly with a vein. Rather, the focus on describing methods with respect to an "artery," "vein," or more broadly a "vessel," is solely for illustrative purposes only.

In any particular method described herein the user gains percutaneous access to the artery, causing a puncture or opening in the artery. In a first embodiment of a method, a wire assembly 2, 102, 202, 302 may be inserted through an opening in a vessel so that a distal end portion of the wire assembly is positioned inside the vessel. The wire assembly 2, 102, 202, 302 is inserted through the vessel with the occlusion member 6, 106, 206, 306 in a collapsed configuration with enough distance such that the wire assembly extends from outside the patient into the artery to provide for an entry guide for other devices. Therefore, once the wire assembly 2, 102, 202, 302 is positioned in the vessel, catheters, introducers, or other devices may are advanced over the wire assembly 2, 102, 202, 302 and through the puncture into the vessel. The wire assembly 2, 102, 202, 302 is advanced through the vessel in a direction opposite of blood flow in the vessel.

The method includes expanding the occlusion member 6, 106, 206, 306 from a collapsed configuration into an expanded configuration such that the occlusion member occludes blood flow in the vessel while the distal end portion of the wire assembly 2, 102, 202, 302 is positioned inside the vessel. The method further includes causing the occlusion member 6, 106, 206, 306 to transition from the expanded configuration into the collapsed configuration so that in the collapsed configuration, the occlusion member 6, 106, 206, 306 may have a cross-sectional dimension relative to a cross-sectional dimension of the shaft 4 that does not substantially inhibit movement of a device along the occlusion member 6, 106, 206, 306 or the shaft 4, as described above.

In a second embodiment of the method, the wire assembly 2, 102, 202, 302 is inserted through a vessel so that a distal end portion of the wire assembly is positioned inside the vessel. In such an embodiment, the method includes coupling a hub to a proximal end portion of the shaft of the wire assembly 2, 102, 202, 302 while the distal end portion is positioned inside the vessel. The method also includes causing the occlusion member 6, 106, 206, 306 to transition from a collapsed configuration into an expanded configuration such that the occlusion member 6, 106, 206, 306 occludes blood flow in the vessel while the distal end portion of the wire assembly 2, 102, 202, 302 is positioned inside the vessel. The method also includes causing the occlusion member 6, 106, 206, 306 to transition from the expanded configuration into the collapsed configuration so that the occlusion member 6, 106, 206, 306 does not occlude blood flow in the vessel.

The embodiments (e.g. the first and second described above) of the methods disclosed herein include other steps and/or functions possible through use of wire assemblies as described herein. For instance, inserting the wire assembly through the opening in the vessel includes advancing the wire assembly 2, 102, 202, 302 opposite a direction of blood flow in the vessel.

In addition, before causing the occlusion member to transition from the expanded configuration into the collapsed configuration, partially collapsing the occlusion member so that the occlusion member does not occlude blood flow in the vessel, and re-expanding the occlusion member so that the occlusion member occludes blood flow in the vessel.

Embodiments of the method may also include, with the occlusion member 6, 106, 206, 306 in the expanded configuration, inserting a device over the shaft 4 while a distal end of the device is positioned inside the vessel and proximal of the occlusion member 6, 106, 206, 306.

Embodiments of the method may also include, with the occlusion member 6, 106, 206, 306 in the collapsed configuration, retracting the wire assembly 2, 102, 202, 302 from the vessel such that the occlusion member 6, 106, 206, 306 does not dilate either the vessel or the opening of the vessel. In an embodiment where the occlusion member 6, 106, 206, 306 is an expandable balloon configured as compliant membrane, expanding the occlusion member 6, 106, 206, 306 from the collapsed configuration into the expanded configuration includes coupling a fluid control device to the proximal end portion of the wire assembly 2, 102, 202, 302 at a connection member. A user may then urge a fluid (e.g. saline or some other biocompatible fluid) in a distal direction through a shaft lumen and into the occlusion member 6, 106, 206, 306.

Embodiments of the method may also include, removing the hub 410 from the shaft 4 while maintaining the occlusion member 6, 106, 206, 306 in the expanded configuration using a valve, and subsequently inserting a device over the shaft 4 and into the vessel. Furthermore, causing the occlusion member 6, 106, 206, 306 to transition from the expanded configuration into the collapsed configuration may also include re-attaching the hub 410 to the proximal end portion of the shaft 4, coupling the fluid control device to the hub 410, and urging fluid from the occlusion member 6, 106, 206, 306 proximally thought the shaft lumen and into the fluid control device.

**In** embodiments of the methods where the occlusion member 6, 106, 206, 306 is an expandable dam configured to occlude blood flow, an actuator can transition the occlusion member 6, 106, 206, 306 between the collapsed configuration and the expanded configuration. In one example, actuation of the actuator pulls the wire in proximal direction causing the occlusion member 6, 106, 206, 306 to 1) compress along the longitudinal axis and 2) expand along a radial direction that is angularly offset with respect to the longitudinal axis.

### Examples

The inventive concepts and uses are further exemplified by way of the following non-limited examples.

One general example includes a method. The method includes inserting a wire assembly through an opening in a vessel so that a distal end portion of the wire assembly is positioned inside the vessel, the wire assembly having a shaft, an occlusion member disposed along a distal end portion of the shaft, and a proximal end portion spaced from the distal end portion along a longitudinal axis, the wire assembly further having cross-sectional dimensions along its length that are perpendicular to the longitudinal axis. The method also includes expanding the occlusion member from a collapsed configuration into an expanded configuration such that the occlusion member occludes blood flow in the vessel while the distal end portion of the wire assembly is positioned inside the vessel. The method also includes causing the occlusion member to transition from the expanded configuration into the collapsed configuration so that in the collapsed configuration, the occlusion member has a cross-sectional dimension relative to a cross-sectional dimension of the shaft that does not substantially inhibit movement of a device along the occlusion member or the shaft.

Implementations may include one or more of the following features. The method may include inserting the wire assembly through the opening in the vessel and advancing the wire assembly opposite a direction of blood flow in the vessel. The cross-sectional dimension of the shaft is any one of about 0.014in (0.0356 cm), 0.018in (0.0457 cm), or 0.035in (0.0889 cm). The cross-sectional dimension of the occlusion member in the collapsed configuration is no greater than about 0.035 in (0.0889 cm). The distal end portion of the shaft includes a preformed j-shape or pigtail shape. The occlusion member is positioned at a distal tip of the shaft. The method may include: before causing the occlusion member to transition from the expanded configuration into the collapsed configuration, partially collapsing the occlusion member so that the occlusion member does not occlude blood flow in the vessel; and re-expanding the occlusion member so that the occlusion member occludes blood flow in the vessel. The method may include, with the occlusion member in the expanded configuration, inserting a device over the shaft until a distal end of the device is positioned inside the vessel and proximal of the occlusion member. The method may include, with the occlusion member in the collapsed configuration, retracting the wire assembly from the vessel such that the occlusion member does not dilate either the vessel or the opening of the vessel.

The occlusion member is an expandable balloon. Expanding the occlusion member from the collapsed configuration into the expanded configuration includes: coupling a fluid control device to the proximal end portion of the wire assembly at a connection member; and urging fluid from the fluid control device in a distal direction through a shaft lumen and into the occlusion member. Coupling the fluid control device to the proximal end portion of the wire assembly includes coupling the fluid control device to a hub that is removably attached to a proximal end portion of the shaft.

The method may include removing the hub from the shaft while maintaining the occlusion member in the expanded configuration using a valve, and subsequently inserting a device over the shaft and into the vessel. Causing the occlusion member to transition from the expanded configuration into the collapsed configuration includes re-attaching the hub to the proximal end portion of the shaft, coupling the fluid control device to the hub, and urging fluid from the occlusion member proximally through the shaft lumen and into the fluid control device.

A cross-sectional dimension of the connection member is not substantially greater than the cross-sectional dimension of the shaft. The occlusion member is an expandable dam. The actuator is configured to transition the occlusion member between the collapsed configuration and the expanded configuration. The actuator includes a tube disposed around and moveable relative to the shaft, where in the collapsed configuration the tube contains the expandable dam, and in the expanded configuration the expandable dam is released from the tube such that expandable dam expands. The expandable dam is a nitinol basket, and the actuator includes a wire coupled to the nitinol basket, where actuation of the actuator pulls the wire in proximal direction causing the nitinol basket to compress along the longitudinal axis and expand along a radial direction that is angularly offset with respect to the longitudinal axis.

The device is a vascular closure device. The method further may include, before expanding the occlusion member from the collapsed configuration into the expanded configuration, deploying an anchor of the vascular closure device inside the vessel. The method may include inserting the wire assembly through the vascular closure device. The cross-sectional dimension of the occlusion member in the collapsed configuration is not substantially greater than the cross-sectional dimension of the shaft.

Another general example includes a method. The method includes inserting a wire assembly through a vessel so that a distal end portion of the wire assembly is positioned inside the vessel, the wire assembly having a shaft and an occlusion member disposed along a distal end portion of the shaft. The method also includes coupling a hub to a proximal end portion of the shaft of the wire assembly while the distal end portion is positioned inside the vessel, where the proximal end portion is spaced from the distal end portion along a longitudinal axis. The method also includes causing the occlusion member to transition from a collapsed configuration into an expanded configuration such that the occlusion member occludes blood flow in the vessel while the distal end portion of the wire assembly is positioned inside the vessel. The method also includes causing the occlusion member to transition from the expanded configuration into the collapsed configuration so that the occlusion member does not occlude blood flow in the vessel.

Implementations may include one or more of the following features. The method may include inserting the wire assembly through the opening in the vessel and advancing the wire assembly opposite a direction of blood flow in the vessel. The method may include before causing the occlusion member to transition from the expanded configuration into the collapsed configuration, partially collapsing the occlusion member so that the occlusion member does not occlude blood flow in the vessel; and re-expanding the occlusion member so that the occlusion member occludes blood flow in the vessel. The method may include, with the occlusion member in the expanded configuration, inserting a device over the shaft until a distal end of the device is positioned inside the vessel and proximal of the occlusion member. The method may include, with the occlusion member in the collapsed configuration, retracting the wire assembly from the vessel such that the occlusion member does not dilate either the vessel or the opening of the vessel.

The occlusion member is an expandable balloon. Expanding the occlusion member from the collapsed configuration into the expanded configuration includes coupling a fluid control device to the proximal end portion of the shaft at a connection member; and urging fluid from the fluid control device in a distal direction through a shaft lumen and into the occlusion member. The wire assembly has cross-sectional dimensions along its length that are perpendicular to the longitudinal axis, and where a cross-sectional dimension of the connection member is not substantially greater than the cross-sectional dimension of the shaft.

The method may include removing the hub from the shaft while maintaining the occlusion member in the expanded configuration using a valve, and subsequently inserting a device over the shaft and into the vessel. Causing the occlusion member to transition from the expanded configuration into the collapsed configuration includes: re-attaching the hub to the proximal end portion of the shaft, coupling the fluid control device to the hub, and urging fluid from the occlusion member proximally thought the shaft lumen and into the fluid control device.

Coupling the fluid control device to the proximal end portion of the shaft includes coupling the fluid control device to the hub. The occlusion member is an expandable dam. The actuator is configured to transition the occlusion member between the collapsed configuration and the expanded configuration. The actuator includes a tube disposed around and moveable relative to the shaft, where in the collapsed configuration the tube contains the expandable dam, and in the expanded configuration the expandable dam is released from the tube such that expandable dam expands to occlude blood flow. The expandable dam is a nitinol basket, and the actuator includes a wire coupled to the nitinol basket, where actuation of the actuator pulls the wire in proximal direction causing the nitinol basket to compress along the longitudinal axis and expand along a radial direction that is angularly offset with respect to the longitudinal axis. The method may include, before expanding the occlusion member from the collapsed configuration into the expanded configuration, deploying an anchor of a vascular closure device inside the vessel. The method may include inserting the wire assembly through the vascular closure device.

Another general example includes a wire assembly configured to occlude a vessel. The wire assembly also includes a shaft that is elongate along a longitudinal axis, the shaft having a proximal end portion, a distal end portion spaced from the proximal end portion along the longitudinal axis, a lumen that extends from the proximal end portion toward the distal end, and a cross-sectional dimension that is perpendicular to the longitudinal axis. The assembly also includes an occlusion member configured to transition between a collapsed configuration and an expanded configuration, where the occlusion member in the collapsed configuration has a cross-sectional dimension that is not substantially greater than a cross-sectional dimension of the shaft.

Implementations may include one or more of the following features. The wire assembly may include a removable hub configured to be coupled to the proximal end portion of the shaft. The connection member is a threaded connector. The connection member is press-fit connector. The wire assembly may include a valve disposed in the lumen of the shaft, the valve configured to permit the occlusion member to maintain the transition from the collapsed configuration to the expanded configuration. In one example, the occlusion member may be an expandable balloon. In another example, the occlusion member may be an expandable dam.

The wire assembly may include an actuator configured to transition the occlusion member between the collapsed configuration and the expanded configuration. The actuator includes a tube disposed around and moveable relative to the shaft, where in the collapsed configuration the tube contains the expandable dam, and in the expanded configuration the expandable dam is released from the tube such that expandable dam expands. The expandable dam is a nitinol basket, and the actuator includes a wire coupled to the nitinol basket, where actuation of the actuator pulls the wire in a proximal direction causing the nitinol basket to compress along the longitudinal axis and expand along a radial direction that is angularly offset with respect to the longitudinal axis.

Another general example includes a wire assembly configured to occlude a vessel. The wire assembly also includes a shaft that is elongate along a longitudinal axis, the shaft having a proximal end portion, a distal end portion spaced from the proximal end portion along the longitudinal axis, and a lumen that extends from the proximal end portion toward the distal end portion. The assembly also includes an occlusion member disposed along the shaft, where the occlusion member is configured to transition between a collapsed configuration and an expanded configuration. The assembly also includes a hub removably coupled to the proximal end portion of the shaft.

Implementations may include one or more of the following features. The occlusion member is positioned at a distal tip of the shaft. The occlusion member is disposed along a step-down region of the shaft. In one example, the connection member is a threaded connector. In another example, the connection member is a press-fit connector. The wire assembly may include a valve disposed in the lumen of the shaft, the valve configured to permit the occlusion member to maintain the transition from the collapsed configuration to the expanded configuration.

The occlusion member may be an expandable balloon. The wire assembly may include a fluid control device couplable to the hub and configured to cause the occlusion member to transition between the collapsed configuration and the expanded configuration. The occlusion member may be an expandable dam. The wire assembly may include an actuator configured to transition the occlusion member between the collapsed configuration and the expanded configuration. The actuator includes a tube disposed around and moveable relative to the shaft, where in the collapsed configuration the tube contains the expandable dam, and in the expanded configuration the expandable dam is released from the tube such that expandable dam expands. The expandable dam is a nitinol basket, and the actuator includes a wire coupled to the nitinol basket, where actuation of the actuator pulls the wire in proximal direction causing the nitinol basket to compress along the longitudinal axis and expand along a radial direction that is angularly offset with respect to the longitudinal axis.

## Claims

1. A wire assembly (2, 102, 202, 302) configured to occlude a vessel, comprising:
a shaft (4) that is elongate along a longitudinal axis (A), the shaft (4) having a proximal end portion (3), a distal end portion (5) spaced from the proximal end portion (3) along the longitudinal axis (A), a lumen that extends from the proximal end portion (3) toward the distal end portion (5), and a cross-sectional dimension (D1) that is perpendicular to the longitudinal axis (A), wherein the distal end portion (5) defines a distal tip of the shaft (4);
an occlusion member (6, 106, 206, 306) positioned along the distal end portion (5) of the shaft (4), the occlusion member (6, 106, 206, 306) configured to transition between a collapsed configuration and an expanded configuration, wherein the occlusion member (6, 106, 206, 306) in the collapsed configuration has a first cross-sectional dimension (D2) that is not greater than the cross-sectional dimension (D1) of the shaft (4) such that the occlusion member (6, 106, 206, 306) is contracted for insertion into the vessel, and the occlusion member (6, 106, 206, 306) in the expanded configuration has a second cross-sectional dimension (D3) that is greater than the first cross-sectional dimension (D2) and the cross-sectional dimension (D1) of the shaft (4);
a removable hub (410) connected to the proximal end portion (3) of the shaft (4); and
a fluid control device coupled to the removable hub (410), the fluid control device configured to transition the occlusion member (6, 106, 206, 306) from the collapsed configuration into the expanded configuration by inflating the occlusion member (6, 106, 206, 306).

2. The wire assembly (2, 102, 202, 302) according to claim 1, further comprising a valve disposed in the lumen of the shaft (4), the valve configured to permit the occlusion member (6, 106, 206, 306) to maintain the transition from the collapsed configuration to the expanded configuration.

3. The wire assembly (2, 102, 202, 302) according to claim 1, wherein the shaft (4) is a guide wire.

4. The wire assembly (2, 102, 202, 302) according to claim 1, wherein the distal end portion (5) has a pigtail shape.

5. The wire assembly (2, 102, 202, 302) according to claim 1, wherein the cross-sectional dimension (D1) of the shaft (4) is no greater than 0.040 in (0.1016 cm).

6. The wire assembly (2, 102, 202, 302) according to claim 1, wherein the shaft (4) includes a step-down region (9) proximal to the distal tip of the shaft (4).

7. The wire assembly (2, 102, 202, 302) according to claim 1, wherein the hub (410) is threadably coupled to the shaft (4) via a threaded connection member.

8. The wire assembly (2, 102, 202, 302) according to claim 1, further comprising a connection member that is a fluid tight connection between the hub (410) and the shaft (4).

9. The wire assembly (2, 102, 202, 302) according to claim 1, wherein the hub (410) and the shaft (4) are coupled via a barbed fitting.

10. The wire assembly (2, 102, 202, 302) according to claim 1, wherein the occlusion member (6, 106, 206, 306) is an expandable balloon comprising a compliant membrane (11) wrapped around and fused into the shaft (4) and configured to be inflated and deflated.

11. The wire assembly (2, 102, 202, 302) according to claim 1, wherein the occlusion member (6, 106, 206, 306) includes a basket coupled to the shaft (4) and a membrane disposed around the basket to facilitate occlusion when inserted in the vessel.

12. The wire assembly (2, 102, 202, 302) according to claim 1, wherein the occlusion member (6, 106, 206, 306) includes an expandable dam (306) having an umbrella shape.

13. The wire assembly (2, 102, 202, 302) according to claim 12, further comprising an actuator configured to transition the occlusion member (6, 106, 206, 306) between the collapsed configuration and the expanded configuration, where the expandable dam (306) is released such that the expandable dam (306) expands.

## Patentansprüche

1. Drahtanordnung (2, 102, 202, 302), die dafür konfiguriert ist, ein Gefäß zu okkludieren, umfassend:
einen Schaft (4), der entlang einer Längsachse (A) länglich ist, wobei der Schaft (4) einen proximalen Endabschnitt (3), einen vom proximalen Endabschnitt (3) entlang der Längsachse (A) beabstandeten distalen Endabschnitt (5), ein Lumen, das sich vom proximalen Endabschnitt (3) in Richtung des distalen Endabschnitts (5) erstreckt, und eine Querschnittsabmessung (D1) aufweist, die senkrecht zur Längsachse (A) ist, wobei der distale Endabschnitt (5) eine distale Spitze des Schafts (4) definiert;
ein Okklusionselement (6, 106, 206, 306), das entlang des distalen Endabschnitts (5) des Schafts (4) positioniert ist, wobei das Okklusionselement (6, 106, 206, 306) dafür konfiguriert ist, zwischen einer kollabierten Konfiguration und einer expandierten Konfiguration überzugehen, wobei das Okklusionselement (6, 106, 206, 306) in der kollabierten Konfiguration eine erste Querschnittsabmessung (D2) aufweist, die nicht größer als die Querschnittsabmessung (D1) des Schafts (4) ist, sodass das Okklusionselement (6, 106, 206, 306) zur Einführung in das Gefäß zusammengezogen ist, und das Okklusionselement (6, 106, 206, 306) in der expandierten Konfiguration eine zweite Querschnittsabmessung (D3) aufweist, die größer als die erste Querschnittsabmessung (D2) und die Querschnittsabmessung (D1) des Schafts (4) ist;
ein abnehmbares Ansatzstück (410), das mit dem proximalen Endabschnitt (3) des Schafts (4) verbunden ist; und
eine Fluidsteuerungsvorrichtung, die mit dem abnehmbaren Ansatzstück (410) gekoppelt ist, wobei die Fluidsteuerungsvorrichtung dafür konfiguriert ist, das Okklusionselement (6, 106, 206, 306) )aus der kollabierten Konfiguration in die expandierte Konfiguration zu überführen, indem das Okklusionselement (6, 106, 206, 306) aufgeblasen wird.

2. Drahtanordnung (2, 102, 202, 302) nach Anspruch 1, ferner umfassend ein Ventil, das im Lumen des Schafts (4) angeordnet ist, wobei das Ventil dafür konfiguriert ist, es dem Okklusionselement (6, 106, 206, 306) zu ermöglichen, den Übergang von der kollabierten Konfiguration in die expandierte Konfiguration aufrechtzuerhalten.

3. Drahtanordnung (2, 102, 202, 302) nach Anspruch 1, wobei der Schaft (4) ein Führungsdraht ist.

4. Drahtanordnung (2, 102, 202, 302) nach Anspruch 1, wobei der distale Endabschnitt (5) eine Pigtail-Form aufweist.

5. Drahtanordnung (2, 102, 202, 302) nach Anspruch 1, wobei die Querschnittsabmessung (D1) des Schafts (4) nicht größer als 0,040 Zoll (0,1016 cm) ist.

6. Drahtanordnung (2, 102, 202, 302) nach Anspruch 1, wobei der Schaft (4) einen verjüngten Bereich (9) proximal zur distalen Spitze des Schafts (4) aufweist.

7. Drahtanordnung (2, 102, 202, 302) nach Anspruch 1, wobei das Ansatzstück (410) über ein Gewindeverbindungselement schraubbar mit dem Schaft (4) gekoppelt ist.

8. Drahtanordnung (2, 102, 202, 302) nach Anspruch 1, ferner umfassend ein Verbindungselement, das eine fluiddichte Verbindung zwischen dem Ansatzstück (410) und dem Schaft (4) ist.

9. Drahtanordnung (2, 102, 202, 302) nach Anspruch 1, wobei das Ansatzstück (410) und der Schaft (4) über ein mit Widerhaken versehenes Anschlussstück gekoppelt sind.

10. Drahtanordnung (2, 102, 202, 302) nach Anspruch 1, wobei das Okklusionselement (6, 106, 206, 306) ein expandierbarer Ballon ist, der eine nachgiebige Membran (11) umfasst, die um den Schaft (4) gewickelt und in diesen eingeschmolzen ist, und dafür konfiguriert ist, aufgeblasen und entleert zu werden.

11. Drahtanordnung (2, 102, 202, 302) nach Anspruch 1, wobei das Okklusionselement (6, 106, 206, 306) ein mit dem Schaft (4) gekoppeltes Körbchen und eine um das Körbchen herum angeordnete Membran aufweist, um die Okklusion bei Einführung in das Gefäß zu erleichtern.

12. Drahtanordnung (2, 102, 202, 302) nach Anspruch 1, wobei das Okklusionselement (6, 106, 206, 306) eine expandierbare Barriere (306) mit einer Schirmform aufweist.

13. Drahtanordnung (2, 102, 202, 302) nach Anspruch 12, ferner umfassend einen Aktuator, der dafür konfiguriert ist, das Okklusionselement (6, 106, 206, 306) zwischen der kollabierten Konfiguration und der expandierten Konfiguration zu überführen, wo die expandierbare Barriere (306) freigegeben wird, sodass sich die expandierbare Barriere (306) expandiert.

## Revendications

1. Ensemble de fils (2, 102, 202, 302) configuré pour occlure un vaisseau, comprenant :
une tige (4) qui s'étend selon un axe longitudinal (A), la tige (4) présentant une partie d'extrémité proximale (3), une partie d'extrémité distale (5) espacée de la partie d'extrémité proximale (3) le long de l'axe longitudinal (A), une lumière qui s'étend de la partie d'extrémité proximale (3) vers la partie d'extrémité distale (5), et une dimension transversale (D1) perpendiculaire à l'axe longitudinal (A), dans lequel la partie d'extrémité distale (5) définit une pointe distale de la tige (4) ;
un élément d'occlusion (6, 106, 206, 306) positionné le long de la partie d'extrémité distale (5) de la tige (4), l'élément d'occlusion (6, 106, 206, 306) étant configuré pour passer d'une configuration repliée à une configuration déployée, dans lequel l'élément d'occlusion (6, 106, 206, 306), dans la configuration repliée, présente une première dimension transversale (D2) qui n'est pas supérieure à la dimension transversale (D1) de la tige (4), de sorte que l'élément d'occlusion (6, 106, 206, 306) est contracté pour l'insertion dans le vaisseau, et dans lequel l'élément d'occlusion (6, 106, 206, 306), dans la configuration déployée, présente une seconde dimension transversale (D3) supérieure à la première dimension transversale (D2) et à la dimension transversale (D1) de la tige (4) ;
un moyeu amovible (410) relié à la partie d'extrémité proximale (3) de la tige (4) ; et
un dispositif de contrôle de fluide couplé au moyeu amovible (410), le dispositif de contrôle de fluide étant configuré pour faire passer l'élément d'occlusion (6, 106, 206, 306)) de la configuration repliée à la configuration déployée en gonflant l'élément d'occlusion (6, 106, 206, 306).

2. Ensemble de fils (2, 102, 202, 302) selon la revendication 1, comprenant en outre une valve disposée dans la lumière de la tige (4), la valve étant configurée pour permettre à l'élément d'occlusion (6, 106, 206, 306) de maintenir l'état résultant du passage de la configuration repliée à la configuration déployée.

3. Ensemble de fils (2, 102, 202, 302) selon la revendication 1, dans lequel la tige (4) est un fil-guide.

4. Ensemble de fils (2, 102, 202, 302) selon la revendication 1, dans lequel la partie d'extrémité distale (5) présente une forme en tire-bouchon.

5. Ensemble de fils (2, 102, 202, 302) selon la revendication 1, dans lequel la dimension transversale (D1) de la tige (4) n'est pas supérieure à 0,040 in (0,1016 cm).

6. Ensemble de fils (2, 102, 202, 302) selon la revendication 1, dans lequel la tige (4) comporte une région à diamètre réduit (9) proximale par rapport à la pointe distale de la tige (4).

7. Ensemble de fils (2, 102, 202, 302) selon la revendication 1, dans lequel le moyeu (410) est couplé de manière filetée à la tige (4) par le biais d'un élément de liaison fileté.

8. Ensemble de fils (2, 102, 202, 302) selon la revendication 1, comprenant en outre un élément de liaison constituant une liaison étanche aux fluides entre le moyeu (410) et la tige (4).

9. Ensemble de fils (2, 102, 202, 302) selon la revendication 1, dans lequel le moyeu (410) et la tige (4) sont couplés par le biais d'un raccord cannelé.

10. Ensemble de fils (2, 102, 202, 302) selon la revendication 1, dans lequel l'élément d'occlusion (6, 106, 206, 306) est un ballon expansible comprenant une membrane déformable (11) enroulée autour de la tige (4) et fusionnée avec celle-ci, et configurée pour être gonflée et dégonflée.

11. Ensemble de fils (2, 102, 202, 302) selon la revendication 1, dans lequel l'élément d'occlusion (6, 106, 206, 306) comporte un panier relié à la tige (4) et une membrane disposée autour du panier pour faciliter l'occlusion lorsqu'il est inséré dans le vaisseau.

12. Ensemble de fils (2, 102, 202, 302) selon la revendication 1, dans lequel l'élément d'occlusion (6, 106, 206, 306) comporte une digue expansible (306) présentant une forme de parapluie.

13. Ensemble de fils (2, 102, 202, 302) selon la revendication 12, comprenant en outre un actionneur configuré pour faire passer l'élément d'occlusion (6, 106, 206, 306) entre la configuration repliée et la configuration déployée, où la digue expansible (306) est libérée de sorte que la digue expansible (306) se déploie.
